# EUROPEAN PATENT APPLICATION

(11) **EP 4 059 446 A1**
(43) Date of publication of application: **21.09.2022**
(21) Application number: 22162600.5
(22) Date of filing: 17.03.2022
(51) Int. Cl.: A61B 17/12, A61B 90/00

(54) **SYSTEMS AND METHODS FOR EMBOLIC IMPLANT DETACHMENT**

(30) Priority: 18.03.2021 US 202117205085
(71) Applicant: DePuy Synthes Products, Inc., Raynham, MA 02767 (US)
(72) Inventor: BLUMENSTYK, David, Raynham, 02767 (US); SOLAUN, Daniel, Raynham, 02767 (US)
(74) Representative: Carpmaels & Ransford LLP

(57) **Abstract**

An aneurysm treatment system can include an embolic implant, a delivery system, and an introducer sheath that are collectively designed so that the combination of the introducer sheath and the delivery system can be used as a deployment apparatus for the embolic implant. The introducer sheath can be moved proximally against an interference feature attached to a pull wire to cause the interference feature to separate from the delivery tube, thereby pulling the pull wire and releasing the implant. Alternatively, the treatment system can include an embolic implant and a delivery system that includes a delivery tube, a proximal extension, a pull wire attached to the proximal extension, and a disconnection feature that can be broken to allow the proximal extension and the pull wire to be translated proximally in relation to the delivery tube.

## Description

### CROSS REFERENCE TO RELATED APPLICATIONS

This application is a continuation in part to prior filed U.S. Patent Application No. 16/218,342 filed on December 12, 2018 which is hereby incorporated by reference as set forth in full herein.

### FIELD OF INVENTION

The present invention generally relates to aneurysm treatment devices and more particularly, to delivery systems for embolic implants.

### BACKGROUND

Numerous intravascular implant devices are known in the field. Many are deployed mechanically, via systems that combine one or more catheters and wires for delivery. Examples of implants that can be delivered mechanically include embolic elements, stents, grafts, drug delivery implants, flow diverters, filters, stimulation leads, sensing leads, or other implantable structures delivered through a microcatheter. Some obstetric and gastrointestinal implants may also be implanted via similar systems that combine one or more catheters and wires. Devices that may be released or deployed by mechanical means vary greatly in design but can employ a similar delivery catheter and wire system. Many such catheter-based delivery systems include a wire for retention of the implant in the catheter until the time for release of the device. These systems are then actuated by retracting or pulling the wire relative to the catheter. Such a wire is referred to herein as a "pull wire".

To pull the pull wire proximally to deploy the implant, a physician can use one of many known deployment apparatuses. Such mechanical deployment apparatuses are typically separate from the delivery system and have moving parts for gripping the pull wire and for moving the pull wire proximally. Deployment methods and apparatuses that do not require auxiliary components and/or complex moving parts can simplify treatment procedures and reduce cost. There is therefore a need for simplified mechanical implant deployment apparatuses.

### SUMMARY

Disclosed herein are various exemplary systems, devices, and methods of the present invention that can address the above needs. Examples can generally include an embolic implantation system that includes an embolic implant, a delivery system, and an introducer sheath that are collectively designed so that the combination of the introducer sheath and the delivery system can be used as a deployment apparatus for the embolic implant. The delivery system can have a pull wire, a delivery tube, and an interference feature attached to the pull wire and positioned at a proximal end of the delivery tube. The introducer sheath can be moved proximally over the delivery tube until it engages the interference feature. To deploy the implant, the introducer sheath can be pressed against the interference feature, causing the interference feature to move proximally in relation to the delivery tube, thereby proximally pulling the pull wire to which the interference feature is attached and deploying the implant.

An example implantation system can include a delivery tube, an embolic coil, an introducer sheath, an interference feature, and an elongated member. The embolic coil can be detachably attached to a distal end of the delivery tube. The interference feature can be positioned at a proximal end of the delivery tube and movable in relation to the delivery tube. The elongated member can be positioned within a lumen of the delivery tube and attached to the interference feature. The introducer sheath can have a lumen sized to slidably receive the delivery tube and the embolic coil, the introducer sheath can be translatable over the delivery tube from the distal end of the delivery tube to the proximal end of the delivery tube, and the introducer sheath can be sized to engage the interference feature. The interference feature can be movable in relation to the delivery tube in response to a force applied by the introducer sheath against the interference feature. The elongated member can be movable in relation to the delivery tube in response to a proximal movement of the interference feature.

The interference feature can be detachable from the delivery tube. The elongated member can be movable to exit the proximal end of the delivery tube in response to a proximal movement of the detached interference feature.

The delivery tube can have a soft section near the distal end of the delivery tube. The length of the embolic coil and the soft section as measured from a distal end of the un-implanted embolic coil to a proximal end of the soft section can be shorter than the end-to-end length of the introducer sheath so that the introducer sheath is sized to fully encompass the un-implanted embolic coil and the soft section. The introducer sheath can be longer than the length of the embolic coil and soft section by about 5 cm.

The system can include a microcatheter, and the delivery tube can have an end-to-end length that is longer than the sum of the end-to-end length of the introducer sheath and an end-to-end length of the microcatheter.

The end-to-end length of the introducer sheath can be between about 46 cm to about 105 cm.

The introducer sheath can be movable from a packaged configuration in which the introducer sheath is positioned to completely encompass the soft section and the embolic coil to a deployment configuration in which the introducer sheath is engaged with the interference feature.

The embolic coil can be detached from the delivery tube by moving the elongated member proximally in relation to the delivery tube.

The interference feature can have a substantially circular surface positioned to engage the proximal end of the introducer sheath.

A distal end of the introducer sheath can be sized to engage a microcatheter to create an enclosed interface through which the embolic coil and at least a portion of the delivery tube can pass.

An example implantation assembly can include a delivery tube, an embolic implant, a pull wire, an engagement bump, and a tubular sheath. The embolic implant can be attached to a distal end of the delivery tube. The pull wire can be disposed within a lumen of the delivery tube and movable to detach the embolic implant from the delivery tube. The engagement bump can be disposed on a proximal end of the pull wire and positioned near a proximal end of the delivery tube. The tubular sheath can be conveyable over the embolic implant and the delivery tube from a distal end of the embolic implant to the proximal end of the delivery tube, and the tubular sheath can be sized to engage the engagement bump. The engagement bump and the pull wire can be movable in relation to the delivery tube in response to a force applied by the tubular sheath to the engagement bump.

The delivery tube can have a soft section extending proximally from the distal end of the delivery tube, and the tubular sheath can measure end-to-end about 5 cm longer than a length measured from a distal end of the embolic implant to a proximal end of the soft section when the embolic implant is attached to the delivery tube and extended in an un-implanted configuration.

The assembly can include a microcatheter, and the delivery tube can have an end-to-end length that is greater than the sum of the length of the introducer sheath and the microcatheter.

The tubular sheath can be movable from a packaged configuration in which the tubular sheath is positioned to completely encompass the soft section and the embolic coil to a deployment configuration in which the tubular sheath is engaged with the engagement bump.

The engagement bump can be detachable from the delivery tube in response to the force applied by the tubular sheath to the engagement bump. The pull wire can be movable to detach the embolic implant from the delivery tube in response to the force applied by the tubular sheath to the engagement bump.

An example method for treating an aneurysm can include the steps of providing an implantation system including an embolic implant, an introducer sheath, a delivery tube, an interference feature, and a pull wire; affixing the pull wire to the interference feature; positioning the pull wire within a lumen of the delivery tube; attaching the interference feature to a proximal end of the delivery tube; attaching the embolic implant at a distal end of the delivery tube; positioning the introducer sheath to encompass the embolic implant and a first portion of the delivery tube; sliding the introducer sheath proximally over the delivery tube; pulling the introducer sheath proximally to apply a force from the introducer sheath to the interference feature; and moving the interference feature and the pull wire proximally in relation to the delivery tube in response to the force.

The first portion of the delivery tube over which the introducer sheath is positioned in the example method can have a soft section. The method can include sizing the introducer sheath to have a length that is greater than the length of the embolic implant and the soft section by about 5 cm. The method can include sizing the introducer sheath to have a length of between about 46 cm to about 105 cm, the length measurable from a distal end to a proximal end of the introducer sheath.

The method can include detaching the embolic implant from the delivery tube in response to moving the interference feature and the pull wire proximally in relation to the delivery tube. The method can include detaching the interference feature from the delivery tube. The interference feature can be detached in response to moving the interference feature and the pull wire proximally in relation to the delivery tube.

The method can include providing a microcatheter; positioning the introducer sheath to engage with the microcatheter while maintaining the embolic implant and the first portion of the delivery tube within the inducer sheath; and translating the embolic implant and the delivery tube distally to position the embolic implant and the first portion of the delivery tube within the microcatheter.

Another example implantation system can include a delivery tube, a proximal extension, an elongated member, an embolic coil, and a disconnection feature. The delivery tube can have a lumen therethrough, a proximal end, and a distal end. The proximal extension can be positioned near the proximal end of the delivery tube. The elongated member can be disposed within the lumen of the delivery tube and can be attached to the proximal extension. The embolic coil can be attached to the delivery tube near the distal end of the delivery tube. The embolic coil can be configured to detach from the delivery tube upon proximal translation of the elongated member. The disconnection feature can fix a position of the proximal extension in relation to the delivery tube. The disconnection feature can be configured to be manipulated to allow proximal translation of the proximal extension and elongated member in relation to the delivery tube.

The disconnection feature can be configured to break apart in response to being manipulated.

The proximal extension can be detachable from the delivery tube. The elongated member can be movable to exit the proximal end of the delivery tube in response to a proximal movement of the detached proximal extension.

The delivery tube can include a distal portion of a hypotube. The proximal extension can include a proximal portion of the hypotube. The disconnection feature can be disposed between the distal portion of the hypotube and the proximal portion of the hypotube.

The disconnection feature can include circumferential laser cut openings in the hypotube.

The disconnection feature can be configured to cause the hypotube to break at the disconnection feature when the proximal extension is bent in relation to the delivery tube.

The proximal extension can include a tube having a lumen therethrough. The elongated member can be disposed within the lumen of the tube of the proximal extension.

Another example implantation assembly can include a delivery tube, a proximal extension, a disconnection feature, an embolic implant, and a pull wire. The delivery tube can have a lumen therethrough, a proximal end, and a distal end. The proximal extension can extend proximally from the proximal end of the delivery tube. The disconnection feature can join the proximal extension to the delivery tube such that a position of the proximal extension is fixed in relation to the delivery tube. The disconnection feature can be configured to open to allow proximal translation of the proximal extension in relation to the delivery tube. The embolic implant can be attached to the distal end of the delivery tube. The pull wire can be disposed within the lumen of the delivery tube and configured to move proximally to detach the embolic implant from the delivery tube when the proximal extension is translated proximally in relation to the delivery tube.

The proximal extension can be detachable from the delivery tube. The pull wire can be movable to exit the proximal end of the delivery tube in response to a proximal movement of the detached proximal extension.

The delivery tube can include a distal portion of a hypotube. The proximal extension can include a proximal portion of the hypotube. The disconnection feature can be disposed between the distal portion of the hypotube and the proximal portion of the hypotube.

The disconnection feature can include circumferential laser cut openings in the hypotube.

The disconnection feature can be configured to cause the hypotube to break at the disconnection feature when the proximal extension is bent in relation to the delivery tube.

The proximal extension can include a tube having a lumen therethrough. The pull wire can be disposed within the lumen of the tube of the proximal extension.

The embolic implant can include an embolic coil.

Another example method for treating an aneurysm can include the steps of manipulating a delivery tube to position an embolic implant attached at a distal end of the delivery tube; disconnecting the delivery tube from a proximal extension extending from a proximal end of the delivery tube; and moving the proximal extension in a proximal direction in relation to the delivery tube, thereby causing a pull wire affixed to the proximal extension to move in the proximal direction in relation to the delivery tube, and thereby causing the embolic implant to detach from the distal end of the delivery tube.

The method can include moving the pull wire to exit the proximal end of the delivery tube in response to moving the proximal extension in the proximal direction in relation to the delivery tube.

The delivery tube can include a distal portion of a hypotube, and the proximal extension can include a proximal portion of the hypotube. The step of disconnecting the delivery tube from the proximal extension can include breaking the hypotube, thereby disconnecting the distal portion of the hypotube from the proximal portion of the hypotube.

The delivery tube can include circumferential laser cut openings between the distal portion of the hypotube and the proximal portion of the hypotube. The step of disconnecting the delivery tube from the proximal extension can include breaking material of the hypotube between the circumferential laser cut openings.

The step of disconnecting the delivery tube from the proximal extension can include bending the hypotube, thereby causing the hypotube to break.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1 is an illustration of an exemplary implantation system according to aspects of the present invention;
FIGs. 2A and 2B are illustrations of an exemplary implantation system such as illustrated in FIG. 1 interfacing with a microcatheter according to aspects of the present invention;
FIGs. 3A through 3C are illustrations of implantation steps that can be performed with an exemplary implantation system such as illustrated in FIG. 1 according to aspects of the present invention;
FIG. 4A illustrates an exemplary implantation system having a breakable disconnection feature according to aspects of the present invention;
FIG. 4B illustrates a cross-sectional view near a proximal end of the exemplary implantation system of FIG. 4A as indicated in FIG. 4A and according to aspects of the present invention;
FIG. 5A illustrates an exemplary implantation system having a twist-lock disconnection feature according to aspects of the present invention;
FIG. 5B illustrates a cross-sectional view near a proximal end of the exemplary implantation system of FIG. 5A as indicated in FIG. 5A and according to aspects of the present invention;
FIG. 6A illustrates an exemplary implantation system having a sliding track according to aspects of the present invention;
FIG. 6B illustrates a cross-sectional view near a proximal end of the exemplary implantation system of FIG. 6A as indicated in FIG. 6A and according to aspects of the present invention;
FIG. 7A illustrates an exemplary implantation system having a stretchable segment according to aspects of the present invention;
FIG. 7B illustrates a cross-sectional view near a proximal end of the exemplary implantation system of FIG. 7A as indicated in FIG. 7A and according to aspects of the present invention;
FIGs. 8A through 8C illustrate cut-away views of an exemplary implantation system having a stretchable segment and a disconnecting feature according to aspects of the present invention;
FIG. 9 illustrates relative dimensions of a delivery system, microcatheter, and introducer sheath as known in the art;
FIGs. 10A through 10C illustrate a sequence of pull wire translation of another exemplary system according to aspects of the present invention; and
FIG. 11 illustrates a close-up view of a perforation disconnection feature according to aspects of the present invention.

### DETAILED DESCRIPTION

Examples presented herein utilize an introducer sheath to facilitate mechanical deployment of an implant. Examples of implants that can be delivered mechanically include embolic elements, stents, grafts, drug delivery implants, flow diverters, filters, stimulation leads, sensing leads, or other implantable structures deliverable through a microcatheter. Some implants are currently packaged with an introducer sheath that is removed from the device and discarded near the beginning of an implantation procedure. For example, in existing systems, embolic coils and other embolic implants can be used to occlude vessels in a variety of medical applications. In many instances, prior to implantation and during handling of an embolic implant outside of a patient, the embolic implant is contained in an introducer sheath. In present treatment practices, once the embolic implant is transferred to a microcatheter, the introducer sheath would be removed from the delivery system and discarded before the embolic implant reaches a treatment site. In examples presented herein, according to the present invention, rather than being discarded, the introducer sheath can be slid proximally and can facilitate deployment of the embolic implant, i.e. detachment of the embolic implant from the delivery system. In order to use the introducer sheath to facilitate deployment, the delivery system can have an interference feature positioned at a proximal end of a delivery tube and attached to a pull wire, and the combination of the introducer sheath, delivery tube, pull wire, and interference feature can be configured such that the introducer sheath can engage with the interference feature and move the interference feature proximally in relation to the delivery tube, thereby pulling the pull wire proximally and deploying the embolic implant. The delivery tube, microcatheter, and introducer sheath can each have a respective length sized such that the introducer sheath can be long enough to cover the embolic implant and sensitive portions of the delivery system, and the delivery tube can be long enough to extend through the entire length of the microcatheter and the entire length of the introducer sheath.

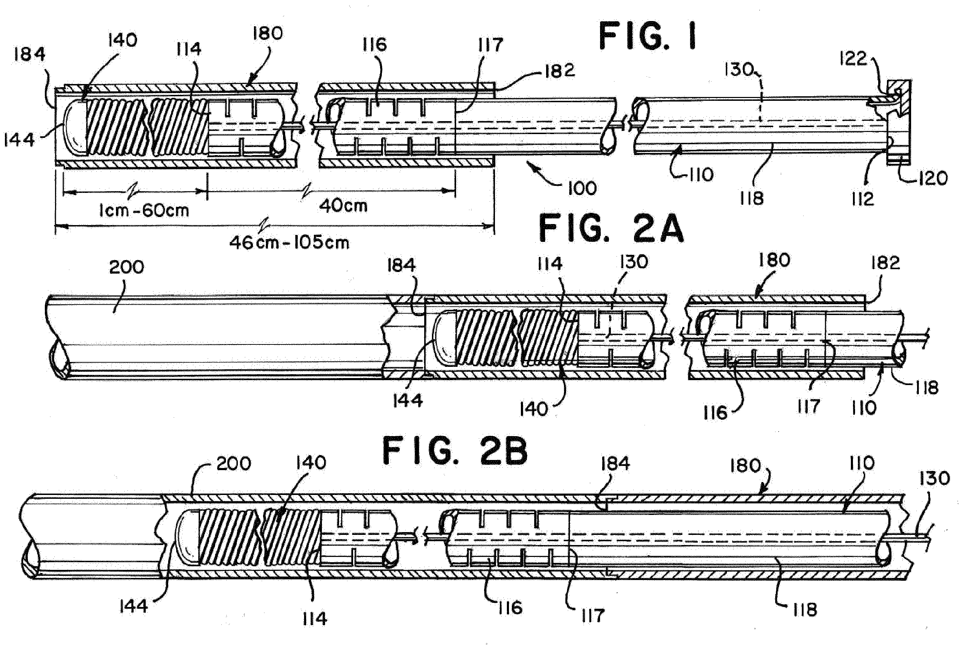

FIG. 1 is an illustration of an exemplary implantation system 100. The implantation system 100 can have an embolic implant 140 such as an embolic coil, embolic braid, or other such implant for filling an aneurysm sac, a delivery tube 110 for delivering the embolic implant 140 to a treatment site, a pull wire 130 disposed within the delivery tube that can be pulled proximally to deploy the embolic implant 140, an interference feature 120 positioned at a proximal end 112 of the delivery tube 110 attached to the pull wire 130 that can be pulled proximally to pull the pull wire 130 proximally, and an introducer sheath 180 that can be moved proximally to engage the interference feature 120 and pull the interference feature 120 proximally.

The introducer sheath 180 can have a lumen therethrough that is sized to slidably receive the delivery tube 110 and the embolic implant 140. The introducer sheath 180 can be sized such that it can be translated proximally from the position illustrated in FIG. 1 over a length of the delivery tube 110 to engage the interference feature 120 positioned at the proximal end 112 of the delivery tube 110.

The interference feature 120 can be movable in relation to the delivery tube 110. For example, the interference feature 120 can be detachably attached to the proximal end 112 of the delivery tube 110, and the system 100 can include a disconnection feature 122 that can be unhooked, torn, broken, twisted, or otherwise manipulated to disconnect the interference feature 120 from the delivery tube 110.

The delivery tube 110 can have a soft section 116 positioned near a distal end 114 of the delivery tube 110 that has a greater flexibility than the remainder (proximal portion) 118 of the delivery tube 110. The embolic implant 140 can be detachably attached to a distal end 114 of the delivery tube 114. The soft section 116 can be designed to allow greater control and stability of the distal end 114 of the delivery tube 110 during implantation and deployment of the embolic implant 140. The soft section 116 can have laser cut notches or groves, and/or the soft section 116 can be made of a more flexible material compared to the remainder 118 of the delivery tube 110.

The introducer sheath 180 can serve the purpose of protecting (packaging) the embolic implant 140 and the soft section 116 of the delivery tube 110 as the system 100 is being handled prior to, and at the beginning of a patient treatment procedure. For this purpose, it is therefore desirable for the introducer sheath 180 to be long enough to completely encompass the embolic implant 140 and the soft section 116 prior to the treatment procedure. The combined length of the embolic implant 140 and the soft section 116 can be measured from a distal end 144 of the embolic implant 140 to a proximal end 117 of the soft section 116. The introducer sheath 180 can have a length measurable from a distal end 184 to a proximal end 182 of the introducer sheath that can be sized a few centimeters longer than the combined length of the embolic implant 140 and the soft section 116 to ensure that the embolic implant 140 and soft section 116 remain protected in case portions of the system 100 shift during handling prior to the treatment procedure. The introducer sheath 180 can have a length that is about 5 cm longer than the combined length of the embolic implant 140 and the soft section 116. For example, the embolic implant 140 can have a length of between about 1 cm and about 60 cm, the soft section 116 can have a length of about 40 cm, and the introducer sheath can have a length that is about 5 cm longer than the sum of the embolic implant 140 length and the soft section 116 length, i.e. between about 46 cm and about 105 cm.

FIGs. 2A and 2B are illustrations of an exemplary implantation system such as illustrated in FIG. 1 interfacing with a microcatheter 200. FIG. 2A illustrates an instant of a treatment procedure near the beginning of the treatment procedure in which an introducer sheath 180 is positioned to cover an embolic implant 140 and a soft portion 116 of a delivery tube 110 in a packaged configuration and a distal end 184 of the introducer sheath 180 is mated or engaged with a proximal end of the microcatheter 200. As shown in FIG. 2A, the distal end 184 of the introducer sheath 180 can be sized to engage the microcatheter 200 to create an enclosed interface through which the embolic implant 140 and the soft portion 116 of the delivery tube 110 can pass. The embolic implant 140 and the delivery tube 110 can be translated distally to push the embolic implant 140 and a portion of the delivery tube 110 into the microcatheter 200.

FIG. 2B illustrates an instant of the treatment procedure in which the embolic implant 140 and the soft portion 116 are positioned within the microcatheter 200. At the instant illustrated in FIG. 2B, the embolic implant 140 and the soft portion 116 are protected by the microcatheter 200 and the introducer sheath can now be pulled proximally 180 or left in place as the delivery tube 110 and embolic implant 140 are further translated distally.

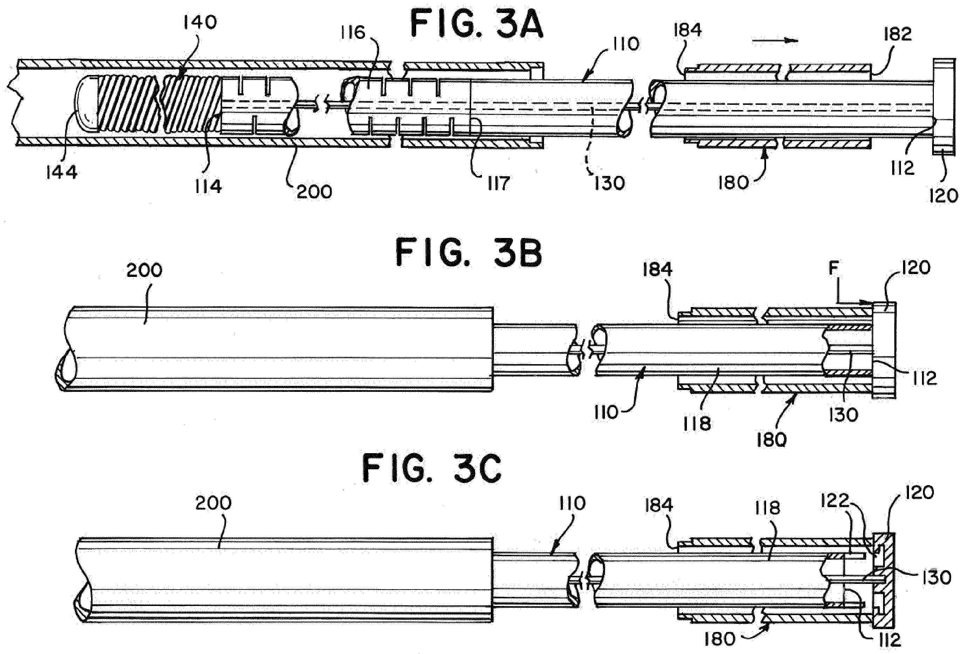

FIGs. 3A through 3C are illustrations of an exemplary implantation system during a series of example implantation steps. FIG. 3A illustrates an embolic implant 140 and a soft portion 116 of a delivery tube 110 positioned inside a microcatheter 200 and an introducer sheath 180 being translated proximally over a proximal portion 118 of the delivery tube 110. As illustrated in FIG. 3A, the introducer sheath 180 can be disengaged from the microcatheter 200 and pulled proximally once the embolic implant 140 and soft section 116 are protected within the microcatheter 200, but before the embolic implant 140 is positioned at a treatment site or within an aneurysm. Alternatively, the introducer sheath 180 can remain engaged to the microcatheter until the embolic implant 140 is positioned at the treatment site or ready to be deployed from the delivery tube 110 and then pulled proximally after the embolic implant 140 is positioned at the treatment site.

FIG. 3B illustrates the introducer sheath 180 in a deployment configuration in which the introducer sheath 180 is engaged with an interference feature 120 positioned near a proximal end 112 of the delivery tube 110. The introducer sheath 180 is shown providing a force F against the interference feature 120. The force can be sufficient to move the interference feature 120 proximally in relation to the delivery tube 110. Prior to the application of the force F, the interference feature 120 can be detachably attached to the proximal end 112 of the delivery tube 110, and the interference feature 120 can be detached from the proximal end 112 of the delivery tube 110 in response to the force F. Alternatively, the interference feature 120 can remain attached to the delivery tube 110 and the force F can be sufficient to move the interference feature 120 in relation to the delivery tube 110.

The introducer sheath 180 can be sized to engage the interference feature 120. As illustrated, the introducer sheath 180 can be tubular and can have a circular proximal end 182, and the interference feature 120 can protrude radially beyond a circumference of the delivery tube 110. The interference feature 120 can be circular, having a circumference larger than a circumference of the proximal end 182 of the introducer sheath 180. The interference feature 120 can provide a flat surface against which the proximal end 182 of the introducer sheath 180 can press. Additionally, or alternatively, the interference feature can have a non-flat surface that can have a slope or a groove for receiving the introducer sheath 180. The interference feature 120 can be a bump positioned near the distal end of the delivery tube that extends beyond the circumference of the delivery tube and extends so that the introducer sheath 180, when slid proximally over the delivery tube 110, must engage the interreference feature 120 before sliding completely over and off the proximal end 112 of the delivery tube 110.

FIG. 3C illustrates the interference feature 120 after being moved proximally in relation to the delivery tube 110 in response to the force F from the introducer sheath 180. The interference feature 120 can be attached to a pull wire 130, and the pull wire 130 can be pulled proximally when the interference feature 180 is moved proximally. The interference feature 120 can be detachably attached to the delivery tube 110 prior to the proximal movement of the interference feature 120, and a detachment feature 122 can be manipulated to facilitate the detachment of the interference feature 120. Once detached, the interference feature 120 can be pulled proximally away from the delivery tube 110, and the pull wire 130 can be moved to exit the proximal end 112 of the delivery tube 110 in response to the pulling of the interference feature 120. The pull wire 130 can be an elongated member that extends through a lumen of the delivery tube 110 toward the embolic implant 140. The pull wire 130 can constitute a component of a deployment system for releasing the embolic implant 140 at the distal end 114 of the delivery tube 110. When the pull wire 130 is pulled proximally, the pull wire 130 can initiate the deployment of the embolic implant 140. The embolic implant 140 can be detached from the delivery tube 110 in response to the proximal movement of the pull wire 130 in relation to the delivery tube 110.

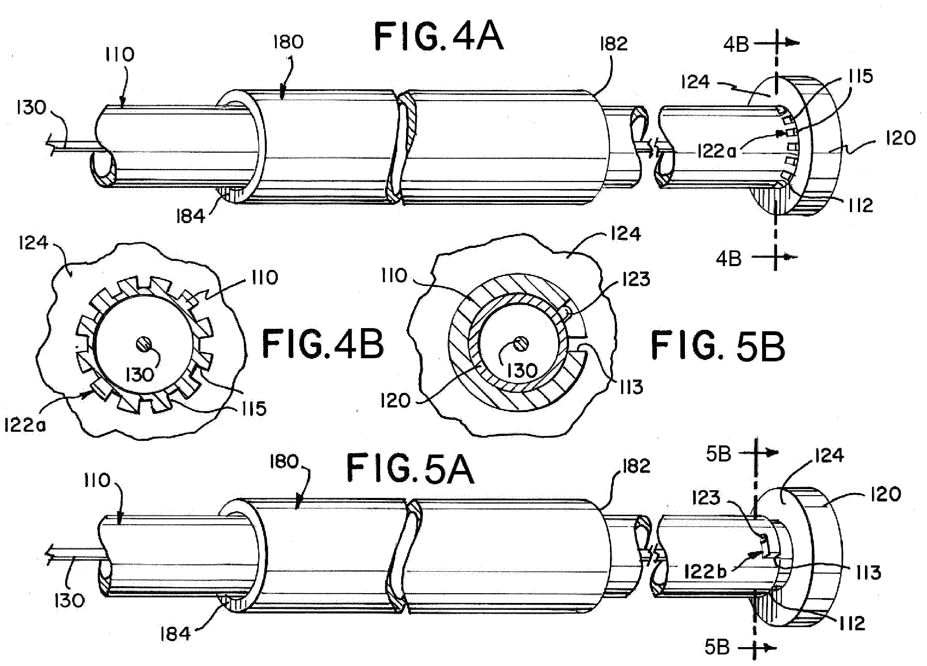

FIG. 4A illustrates an exemplary implantation system having a breakable disconnection feature 122a. The implantation system can have an interference feature 120 detachably attached to a delivery tube 110 by the breakable disconnection feature 122a. The delivery tube 110 can include notches 115 that are areas in which material is removed from the delivery tube 110. The notches 115 can be positioned at a proximal end 112 of the delivery tube 110. The proximal end 112 of the delivery tube 110 can be attached to the interference feature 120 by gluing, welding, or other means. The notches 115 can be a breakable section 122a of the delivery tube 110. When an introducer sheath 180 is pressed against the interference feature 120, a force from the interference feature 120 can cause the breakable section 122a to break, and the interference feature 120 can then be moved proximally in relation to the delivery tube 110. The interference feature 120 can have a circular surface 124 against which the introducer sheath 180 can press. FIG. 4B is a cross-sectional view near a proximal end of the exemplary implantation system as indicated in FIG. 4A.

FIG. 5A illustrates an exemplary implantation system having a twist-lock disconnection feature 122b. The implantation system can have a delivery tube 110 with groove 113 cut at a proximal end 112 and an interference feature 120 that has a bump 123 or another feature that can engage the groove 113. The interference feature 120 can be detachably attached to the delivery tube 110 by the twist-lock disconnection feature (and/or a bayonet connector) 122b. The interference feature 120 can extend within a lumen of the delivery tube 110 at the proximal end 112 of the delivery tube 110 and have a bump or protrusion 123 that can be positioned in the groove 113 in the delivery tube 110 to maintain the attachment between the interference feature 120 and the delivery tube 110. The bump or protrusion 123 can be slid through the groove 113 to detach the interference feature 120 from the delivery tube 110. The groove 113 can be L shaped, and the interference feature 120 can be twisted in relation to the delivery tube 110 and then pulled proximally in relation to the delivery tube 110 to disconnect the twist-lock disconnection feature 122b. FIG. 5B illustrates a cross-sectional view near a proximal end of the exemplary implantation system as indicated in FIG. 5A.

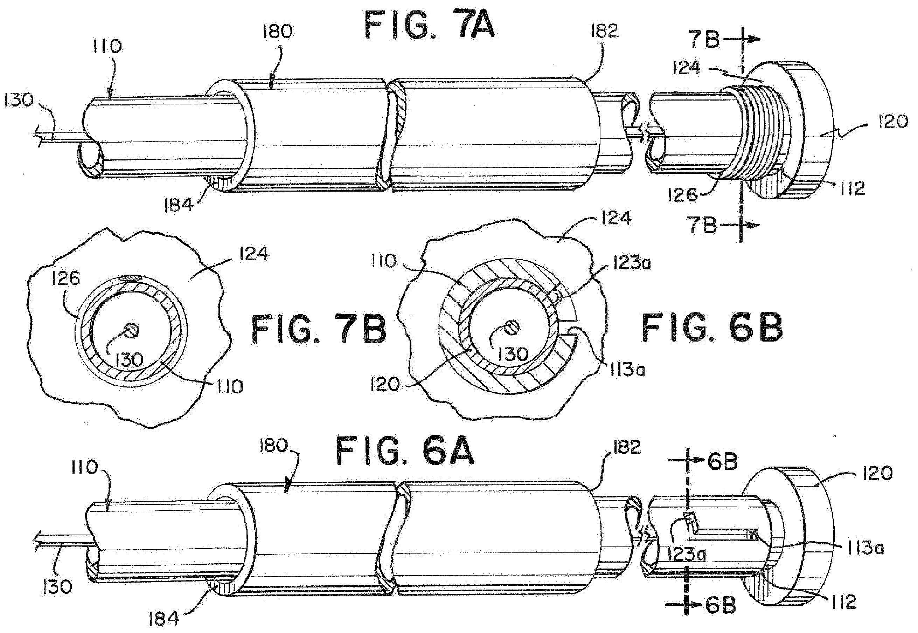

While FIGs. 4A through 5B illustrate examples of an interference feature 120 that is movable in relation to the delivery tube 110 after detaching from the delivery tube 110, the interference feature need not be detached, and can be movable in relation to the delivery tube 110 without detaching. FIGs. 6A through 8C illustrate example systems wherein the interference feature 120 remains at least partially attached to the delivery tube 110.

FIG. 6A illustrates an exemplary implantation system having a sliding track 113a and a bump or protrusion 123a. The sliding track 113a can be cut from a portion of the delivery tube 110 near the proximal end 112 of the delivery tube 110. The interference feature 120 can have an engagement bump or protrusion 123a that is positioned to slide within the track 113a. The track 113a can extend along a portion of a length of the delivery tube 110, and the bump 123a can slide within the track 113a, allowing the interference feature 120 to move in a proximal direction in relation to the delivery tube 110. The track 113a can be L shaped, and the interference feature 120 can be twisted in relation to the delivery tube 110 and then pulled proximally in relation to the delivery tube 110 to move the interference feature 120 in relation to the delivery tube 110. The interference feature 120 can be attached to a pull wire 130, and the movement of the interference feature 120 can move the pull wire 130 to deploy an embolic implant 140.

FIG. 6B illustrates a cross-sectional view near a proximal end of the exemplary implantation system as indicated in FIG. 6A.

FIG. 7A illustrates an exemplary implantation system having a stretchable segment 126. The implantation system can have a delivery tube 110 with a stretchable segment 126 positioned near a proximal end 112 of the delivery tube 110. The stretchable segment 126 can be a region of the delivery tube 110 that has a propensity to stretch in response to a force that creates tension along a length of the delivery tube 110 that includes the stretchable segment 126. The stretchable segment 126 can include a coil that is compressed in an initial state as illustrated in FIG. 7A, a laser cut portion of the tube, and/or a portion of tubing having greater elasticity. The stretchable segment 126 can extends in response to a force provided by the introducer sheath 180 against the interference feature 120. The stretchable segment 126 can allow the pull wire and the interference feature 120 to move proximally in relation to the delivery tube 110 without the interference feature 120 becoming disconnected from the delivery tube 110. The stretchable segment 126 can have a fully extended length that is determined by the material properties and/or construction of the stretchable segment 126. The fully extended length can limit the distance that the interference feature 120 can be moved proximally in relation to the delivery tube 110. FIG. 7B illustrates a cross-sectional view near a proximal end of the exemplary implantation system as indicated in FIG. 7A.

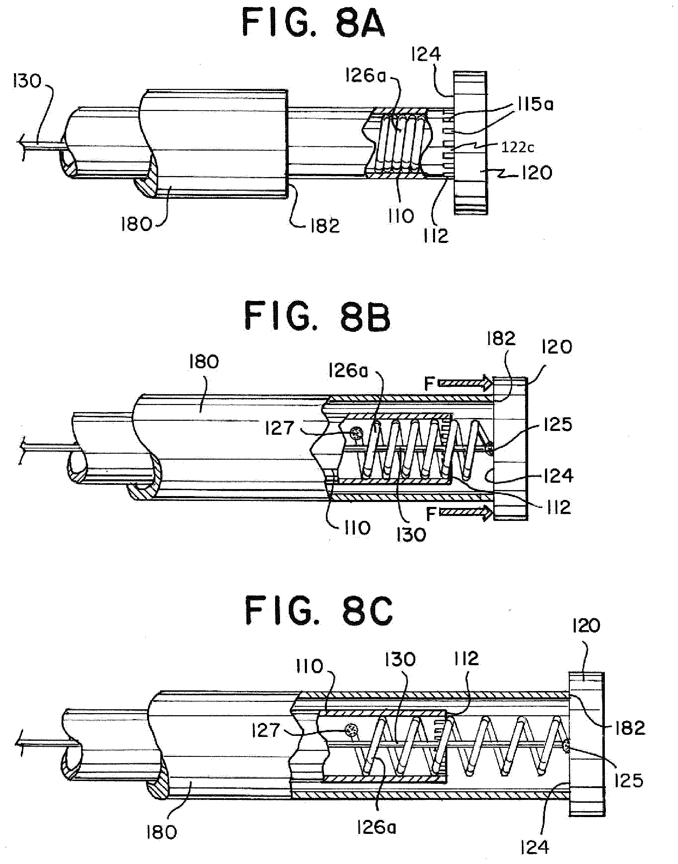

FIGs. 8A through 8C illustrate an exemplary implantation system having a stretchable element 126a and a detachment feature 122c. The disconnection feature 122c illustrated in FIGs. 8A through 8C can include notches 115B in a delivery tube 110, similar to that illustrated in FIGs. 4 and 4B. It is contemplated that other disconnection features, including the disconnection features illustrated in, and described in relation to FIGs. 1, 3A-3C, 5, or 5B could be combined with a stretchable segment like those described herein or otherwise known. When used in combination with the stretchable segment 126a, the disconnection feature 122c can be positioned along a length of the delivery tube 110 at or near the stretchable segment 126a. Both the stretchable segment 126a and the disconnection feature 122c can be positioned near the proximal end 112 of the delivery tube 110.

FIG. 8A illustrates the stretchable element 126a positioned within a lumen of the delivery tube 110 near the proximal end 112 of the delivery tube 110. In FIG. 8A, the delivery tube 110 is illustrated cut-away to show coils of the stretchable element 126a within. In the configuration illustrated in FIG. 8A, interference feature 120 can be attached to the delivery tube 110 via the stretchable element 126a and the detachment feature 122c.

FIG. 8B illustrates an introducer sheath 180 moved proximally to engage the interference feature 120, break the detachment feature 122c, and begin to stretch the stretchable element 126a. The delivery tube 110 and the introducer sheath 180 are shown cut-away. A pull wire 130 can be positioned within the delivery tube 110. The pull wire 130 can be pulled proximally as the interference feature 120 is moved proximally. The stretchable element 126a can be attached to the interference feature 120 with a weld, adhesive, or other connection 125. The stretchable element 126a can be attached to the delivery tube 110 with a weld, adhesive, or other connection 127. After the detachment feature 122c is detached, the stretchable element 126a can maintain an attachment between the interference feature 120 and the delivery tube 110.

FIG. 8C illustrates the introducer sheath 180 moved further proximally to move the interference feature 120 and pull wire 130 further proximally and further stretch the stretchable element 126a. The stretchable element 126a can have a fully extended length that is determined by the material properties and/or construction of the stretchable element 126a. The fully extended length can limit the distance that the interference feature 120 can be moved proximally in relation to the delivery tube 110.

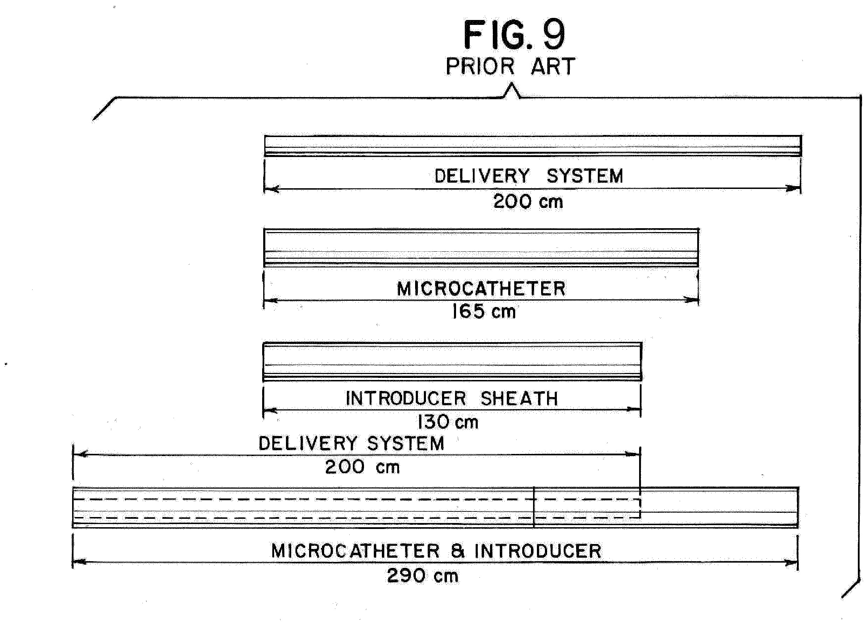

FIG. 9 illustrates relative dimensions of a delivery system, microcatheter, and introducer sheath as known in the art. Known delivery systems are typically 200 cm long, known microcatheters are typically 165 cm long, and known introducer sheaths are typically 130 cm long. In known practices, the introducer sheath is typically removed after an embolic implant and any sensitive portions of the delivery system are inserted into the microcatheter. According to known practices, an introducer sheath cannot remain around the delivery system during the deployment step of the embolic implant because the combined length of known microcatheters and introducers is several centimeters longer than known delivery systems. It is an aspect of the present invention to size a delivery system and an introducer sheath so that the introducer sheath can remain on the delivery system through the embolic implant deployment step. In example systems presented herein, an implantation system can include a delivery system, microcatheter, and introducer sheath, wherein the delivery system is longer than the combined length of the microcatheter and the introducer sheath.

An aneurysm can be treated with an implantation system such as any of the implantation systems disclosed herein in relation to the present invention by executing some or all the following steps, not necessarily in order. An implantation system 100 having an embolic implant 140, an introducer sheath 180, a delivery tube 110, an interference feature 120, and a pull wire 130 can be provided. The pull wire 130 can be affixed to the interference feature 120. The pull wire 130 can be positioned within a lumen of the delivery tube 110. The interference feature 120 can be attached to a proximal end 112 of the delivery tube 110. The embolic implant 140 can be attached at a distal end 114 of the delivery tube 110. The introducer sheath 180 can be positioned to encompass the embolic implant 140 and a first portion of the delivery tube 110. The first portion of the delivery tube 110 can comprise a soft section 116. The introducer sheath can be sized to have an end-to-end length that is longer by about 5 cm than a length measurable from a distal end 144 of the embolic implant 140 to a proximal end 117 of the soft section 116. The introducer sheath 180 can be sized so that the end-to-end length is between about 46 cm and about 105 cm. A microcatheter 200 can be provided. The introducer sheath 180 can be positioned to engage with the microcatheter 200 while maintaining the embolic implant 140 and the first portion of the delivery tube 110 within the introducer sheath 180. The embolic implant 140 and the delivery tube 110 can be translated distally to position the embolic implant 140 and the first portion of the delivery tube 110 within the microcatheter 200. The introducer sheath 180 can be slid proximally over the delivery tube 110. The introducer sheath 180 can be pulled proximally to apply a force from the introducer sheath 180 to the interference feature 120. The interference feature 120 and the pull wire 130 can be moved proximally in relation to the delivery tube 110 in response to the force. The interference feature 120 can be detached from the delivery tube 110. The embolic implant 140 can be detached from the delivery tube 110 in response to moving the interference feature 120 and the pull wire 130 proximally in relation to the delivery tube 110.

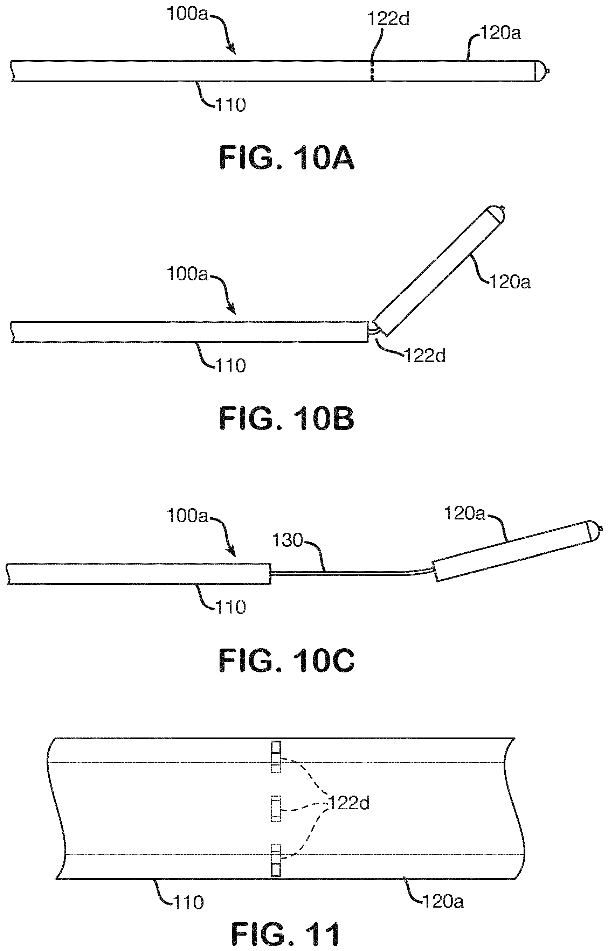

FIGs. 10A through 10C are illustrations of another exemplary implantation system 100a which has a proximal extension 120a that functions similarly to the interference features 120 illustrated elsewhere herein with an exception that the proximal extension 120a can be manipulated by hand without the use of the introducer sheath 180. The implantation system 100a can have an embolic implant 140 such as an embolic coil, embolic braid, or other such implant for filling an aneurysm sac, a delivery tube 110 for delivering the embolic implant 140 to a treatment site, and a pull wire 130 disposed within the delivery tube that can be pulled proximally to deploy the embolic implant 140.

FIG. 10A illustrates the system 100a in a delivery configuration. While the system 100a is in the delivery configuration, the delivery tube 110 can be introduced through an introducer sheath 180, the introducer sheath 180 can be discarded, and the embolic implant 140 can be positioned at a treatment site.

FIG. 10B illustrates the system 100a at step in preparation for deploying the embolic implant 140. The system 100a can include a disconnection feature 122d between the delivery tube 110 and the proximal extension 120a that can be manipulated to separate the delivery tube 110 from the proximal extension 120a. As illustrated, the disconnection feature 122d can be broken by bending the proximal extension 120a in relation to the delivery tube 110.

FIG. 10C illustrates the proximal extension 120a being pulled proximally to thereby pull the pull wire 130 proximally and release the embolic implant 140.

FIG. 11 illustrates a close-up view of the disconnection feature 122d. As illustrated, the delivery tube 110 and the proximal extension 120a be formed from a contiguous hypotube. The disconnection feature 122d can include laser-cut features in the hypotube that are sized and spaced so that stress is concentrated on mater between the laser-cut features so that the hypotube breaks at the disconnection feature 122d when bent as illustrated in FIG. 10B. The laser-cut features can be alternatively sized, shaped, and otherwise configured to concentrate stress on hypotube material so that the delivery tube 110 breaks at a predetermined position in response to bending.

The disconnection feature can alternatively be configured similar to other disconnection features 122a-c illustrated herein, variations thereof, and alternatives thereto as understood by a person skilled in the pertinent art. For instance, the disconnection feature 122d of the system 100a illustrated in FIGs. 10A through 11 can be configured similarly to the disconnection feature 122b illustrated in FIG. 5A or a variation thereof that includes twisting the proximal extension 120a in relation to the delivery tube 110. Although not illustrated, the system 100a can include a stretchable sleeve attached to the delivery tube 110 and the proximal extension 120a that is configured to stretch when the proximal extension 120a is pulled proximally as illustrated in FIG. 10C.

The descriptions contained herein are examples of embodiments of the invention and are not intended in any way to limit the scope of the invention. As described herein, the invention contemplates many variations and modifications of the implantation system and associated methods, including alternative geometries of system components, alternative materials, additional or alternative method steps, etc. Modifications apparent to those skilled in the pertinent art are intended to be within the scope of the claims which follow.

Aspects of the invention:
1. A method for treating an aneurysm comprising:
   manipulating a delivery tube to position an embolic implant attached at a distal end of the delivery tube;
   disconnecting the delivery tube from a proximal extension extending from a proximal end of the delivery tube; and
   moving the proximal extension in a proximal direction in relation to the delivery tube, thereby causing a pull wire affixed to the proximal extension to move in the proximal direction in relation to the delivery tube, and thereby causing the embolic implant to detach from the distal end of the delivery tube.
2. The method of aspect 1, further comprising:
   moving the pull wire to exit the proximal end of the delivery tube in response to moving the proximal extension in the proximal direction in relation to the delivery tube.
3. The method of aspect 1,
   wherein the delivery tube comprises a distal portion of a hypotube,
   wherein the proximal extension comprises a proximal portion of the hypotube, and
   wherein disconnecting the delivery tube from the proximal extension further comprises breaking the hypotube thereby disconnecting the distal portion of the hypotube from the proximal portion of the hypotube.
4. The method of aspect 3,
   wherein the delivery tube comprises circumferential laser cut openings between the distal portion of the hypotube and the proximal portion of the hypotube, and
   wherein disconnecting the delivery tube from the proximal extension further comprises breaking material of the hypotube between the circumferential laser cut openings.
5. The method of aspect 3, wherein disconnecting the delivery tube from the proximal extension further comprises bending the hypotube, thereby causing the hypotube to break.

## Claims

1. An implantation system comprising:
a delivery tube comprising a lumen therethrough, a proximal end, and a distal end;
a proximal extension positioned approximate the proximal end of the delivery tube;
an elongated member disposed within the lumen of the delivery tube and attached to the proximal extension;
an embolic coil attached to the delivery tube approximate the distal end of the delivery tube and configured to detach from the delivery tube upon proximal translation of the elongated member; and
a disconnection feature fixing a position of the proximal extension in relation to the delivery tube and configured to be manipulated to allow proximal translation of the proximal extension and elongated member in relation to the delivery tube.

2. The system of claim 1, wherein the disconnection feature is configured to break apart in response to being manipulated.

3. The system of claim 1,
wherein the proximal extension is detachable from the delivery tube, and
wherein the elongated member is movable to exit the proximal end of the delivery tube in response to the proximal translation of the proximal extension.

4. The system of claim 1, wherein the delivery tube comprises a distal portion of a hypotube, the proximal extension comprises a proximal portion of the hypotube, and the disconnection feature is disposed between the distal portion of the hypotube and the proximal portion of the hypotube.

5. The system of claim 4, wherein the disconnection feature comprises circumferential laser cut openings in the hypotube.

6. The system of claim 4, wherein the disconnection feature is configured to cause the hypotube to break at the disconnection feature when the proximal extension is bent in relation to the delivery tube.

7. The system of claim 1,
wherein the proximal extension comprises a tube comprising a lumen therethrough, and
wherein the elongated member is disposed within the lumen of the tube of the proximal extension.

8. An implantation assembly comprising:
a delivery tube comprising a lumen therethrough, a proximal end, and a distal end;
a proximal extension extending proximally from the proximal end of the delivery tube;
a disconnection feature joining the proximal extension to the delivery tube such that a position of the proximal extension is fixed in relation to the delivery tube, the disconnection feature being configured to open to allow proximal translation of the proximal extension in relation to the delivery tube;
an embolic implant attached to the distal end of the delivery tube; and
a pull wire disposed within the lumen of the delivery tube and configured to move proximally to detach the embolic implant from the delivery tube when the proximal extension is translated proximally in relation to the delivery tube.

9. The assembly of claim 8, wherein the proximal extension is detachable from the delivery tube and wherein the pull wire is movable to exit the proximal end of the delivery tube in response to the proximal translation of the proximal extension in relation to the delivery tube.

10. The assembly of claim 8, wherein the delivery tube comprises a distal portion of a hypotube, the proximal extension comprises a proximal portion of the hypotube, and the disconnection feature is disposed between the distal portion of the hypotube and the proximal portion of the hypotube.

11. The assembly of claim 10, wherein the disconnection feature comprises circumferential laser cut openings in the hypotube.

12. The assembly of claim 10, wherein the disconnection feature is configured to cause the hypotube to break at the disconnection feature when the proximal extension is bent in relation to the delivery tube.

13. The assembly of claim 8,
wherein the proximal extension comprises a tube comprising a lumen therethrough, and
wherein the pull wire is disposed within the lumen of the tube of the proximal extension.

14. The assembly of claim 8, wherein the embolic implant comprises an embolic coil.
